(19) Europäisches Patentamt
European Patent Office
Office européen des brevets

(11) Publication number: **0 066 351**
**B1**

(12) **EUROPEAN PATENT SPECIFICATION**

(45) Date of publication of patent specification: **26.06.85**

(51) Int. Cl.[4]: **C 07 C 93/14,** C 07 C 91/30, C 07 C 89/02, A 61 K 31/135

(21) Application number: **82301019.4**

(22) Date of filing: **01.03.82**

(54) **Arylethanol amine derivatives, their preparation and use in pharmaceutical compositions.**

(30) Priority: **06.03.81 GB 8107050**
**11.07.81 GB 8121443**

(43) Date of publication of application:
**08.12.82 Bulletin 82/49**

(45) Publication of the grant of the patent:
**26.06.85 Bulletin 85/26**

(84) Designated Contracting States:
**BE CH DE FR GB IT LI NL SE**

(56) References cited:
**EP-A-0 007 204**
**EP-A-0 023 385**
**EP-A-0 040 000**

**Chemical Abstracts vol. 86, no. 17, 25 April 1977, Columbus, Ohio, USA, S.L. BEREGI et al. "Structure-anorectic activity relations in substituted phenethylamines" page 16, column 1, abstract no. 114995j**

(73) Proprietor: **BEECHAM GROUP PLC**
**Beecham House Great West Road**
**Brentford Middlesex TW8 9BD (GB)**

(72) Inventor: **Cantello, Barrie Christian Charles**
**8 Woodside Way**
**Redhill Surrey (GB)**
Inventor: **Hindley, Richard Mark**
**19 Cockshot Road**
**Reigate Surrey (GB)**

(74) Representative: **Russell, Brian John et al**
**European Patent Attorney Beecham Pharmaceuticals Great Burgh Yew Tree Bottom Road**
**Epsom Surrey, KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to derivatives of ethanolamine which have anti-hyperglycaemic and/or anti-obesity and/or anti-inflammatory and/or platelet aggregation inhibition activity, to processes for their preparation and to their use in medicine. These activities are coupled with low cardiac stimulation activity for certain of the derivatives.

Belgian Patent Specification No 843422 discloses compounds of the formula (I):

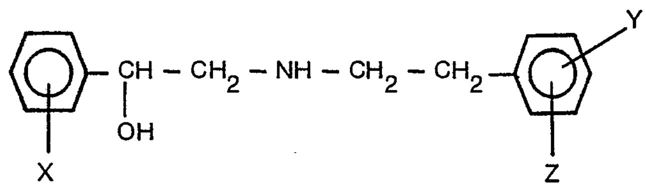

(I)

in which Y is hydrogen, methoxy, or ethoxy, Z is methoxy or ethoxy, and X is hydrogen, chlorine, bromine, fluorine, methyl or trifluoromethyl. These compounds are disclosed as intermediates in the preparation of other compounds for treating Parkinson's disease. The intermediates themselves are not disclosed as having pharmaceutical activity in their own right.

UK Patent Specification No 1574208 also discloses compounds of formula (I) wherein Y is hydrogen, or methoxy, Z is methoxy, and X is hydrogen, chlorine, bromine, fluorine, methyl, trifluoromethyl or methoxy. These compounds are also disclosed as intermediates in the preparation of other compounds for treating Parkinson's disease, and are not disclosed as having pharmaceutical activity.

We have now discovered a group of compounds, some of which fall within the scope of formula (I) but none are specifically disclosed in Belgian No. 843422 or UK No. 1574208, which have anti-hyperglycaemic and/or anti-obesity and/or anti-inflammatory and/or platelet aggregation inhibition activity. These activities could not have been predicted from the aforementiopned patent specifications.

According to the present invention there is provided a compound of formula (II):

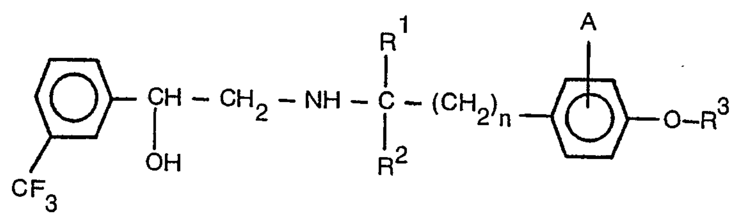

(II)

or a salt thereof;
wherein

$R_1$ is hydrogen or methyl;

$R_2$ is hydrogen or methyl;

n is 1 or 2;

$R_3$ is hydrogen, $C_{1-12}$ alkyl, $C_{1-10}$ cycloalkyl or benzyl optionally substituted with $C_{1-6}$ alkyl, $C_{1-6}$ alkoxy, or halogen;

and

A is hydrogen, halogen, hydroxy, $C_{1-6}$ alkyl or $C_{1-6}$ alkoxy.

The term $C_{1-12}$ alkyl is used herein to include a straight or branched alkyl group, optionally substituted by cycloalkyl, such that the total number of carbon atoms in the $R_3$ group is no more than twelve.

Preferably n is 1.

Preferably $R^1$ is hydrogen and $R^2$ is methyl.

Preferred alkyl groups for $R^3$ are $C_{1-6}$ alkyl, suitably $C_{1-4}$ alkyl.

Pharmaceutically acceptable salts of compounds of formula (II) include acid addition salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicyclic acid or acetylsalicyclic acid.

In the case where $R^3$ is hydrogen, the salts may include alkali metal and alkaline earth metal salts.

The salts of compounds of formula (II) need not be pharmaceutically acceptable as they are also useful in the preparation of other compounds of formula (II) and in the separation of stereoisomers of compounds of formula (II) when the salt ion is also optically active.

The salts of compounds of formula (II) may be produced by treating the compound of formula (II) with the appropriate acid.

Compounds of formula (II) have at least one asymmetric carbon atom, ie the one bearing the hydroxyl and m-trifluoromethyl phenyl group, and, when $R^1$ and $R^2$ are different, the carbon atom bearing $R^1$ and $R^2$ is also asymmetric. The compounds may, therefore, exist in at least two and often four stereoisomeric forms. The present invention encompasses all stereoisomers of the compounds of formula (II) whether free from other stereoisomers or admixed with other stereoisomers in any proportion and thus includes, for instance, racemic mixtures of enantiomers.

Preferably, the carbon atom bearing the hydroxyl and m-trifluoromethyl substituted phenyl group has the R configuration.

The most potent compounds of formula (II) are those wherein $R^1$ and $R^2$ are different and both asymmetric carbon atoms are in the R configuration.

The absolute configuration of any compound of formula (II) may be determined by conventional X-ray crystallographic techniques.

It is believed that, in the $^{13}$Cnmr of compounds of formula (II) wherein one of $R^1$ and $R^2$ is hydrogen and the other is methyl, the diasteroisomer having the greater anti-obesity and anti-hyperglycaemic activity is that for which the signal of the methyl group carbon atom appears at higher field (the lower numerical value when expressed in ppm) in $d_6$DMSO solution. The paired resonances often appear at approximately 20 ppm (less active) and slightly below 20 ppm (more active) down field from tetramethylsilane. Other paired resonances can occur for the carbon atoms attached directly to the nitrogen atom and the carbon which carries the hydroxyl and phenyl groups. Again the paired resonance of the more active diastereoisomer of the investigated compounds appear at the higher field position.

The present invention also provides a process for producing a compound of formula (II) or a salt thereof, which process comprises reducing an oxo-group and/or a double bond in a comound of formula (III):

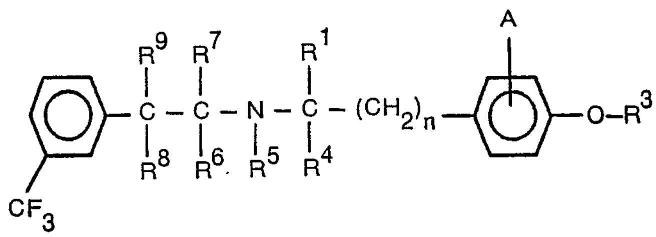

(III)

wherein

$R^1$, $R^3$, A and n are as defined in relation to formula (II);

$R^4$ is a group $R^2$ as defined in relation to formula (II) or together with $R^5$ forms a bond;

$R^5$ is hydrogen or together with $R^4$ or $R^6$ forms a bond;

$R^6$ is hydrogen or together with $R^7$ forms an oxo-group or together with $R^5$ forms a bond;

$R^7$ is hydrogen or together with $R^6$ forms an oxo-group;

$R^8$ is hydroxyl or together with $R^9$ forms an oxo-group;

$R^9$ is hydrogen or together with $R^8$ forms an oxo-group, provided that there is no more than one oxo-group and optionally thereafter forming a salt of the compound of formula (II) so formed.

The aforementioned reduction may be effected by conventional chemical or catalytic methods, such as chemical reduction using lithium aluminium hydride, sodium cyanoborohydride or sodium borohydride or by catalytic hydrogenation using catalysts such as palladium on charcoal, or platinum, for instance, as platinum oxide.

Reduction by sodium borohydride is conveniently effected in a lower alkanolic solvent such as methanol. The reaction is generally carried out at from 0—20°C.

Reduction by lithium aluminium hydride is conveniently effected in a dry, ether solvent such as diethyl ether or tetrahydrofuran at ambient or elevated temperature.

Catalytic reduction is conveniently effected in a conventional hydrogenation solvent such as a lower alkanol, for instance ethanol. The hydrogenation is generally carried out under hydrogen gas at about 1 atmosphere pressure [1.03 $Kgcm^{-2}$] to about 10 atmospheres pressure [10.3 $Kgcm^{-2}$] and at ambient or elevated temperature.

Preferred process aspects of the invention for producing compounds of formula (II) comprise

(a) reducing a compound of formula (IIIA):

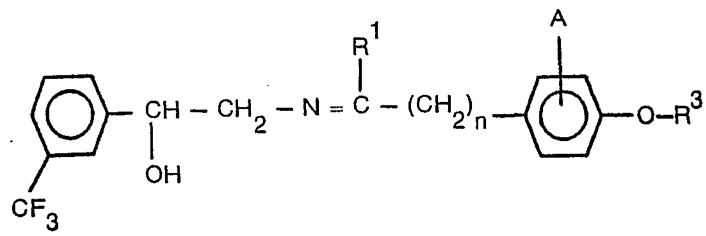

(IIIA)

or (b) reducing a compound of formula (IIIB):

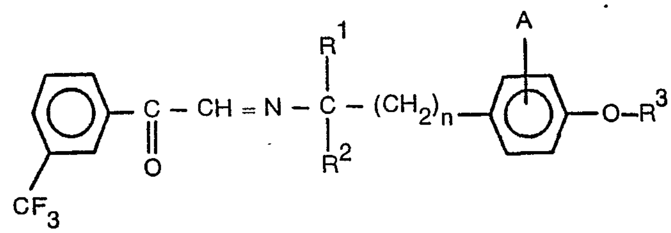

(IIIB)

or (c) reducing a compound of formula (IIIC):

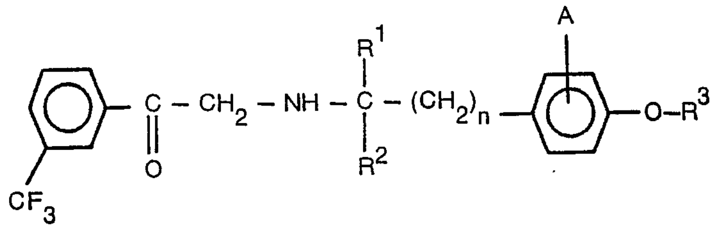

(IIIC)

or its N-benzyl derivative,
or (d) reducing a compound of formula (IIID):

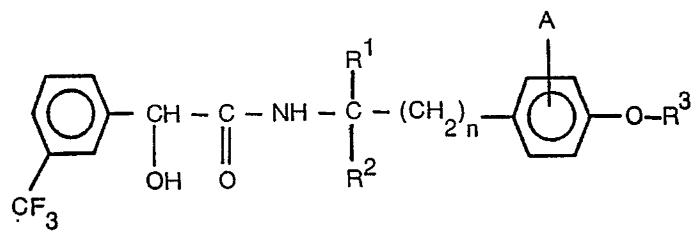

(IIID)

wherein $R^1$, $R^2$, $R^3$, A and n, are as defined in relation to formula (II).

A particularly preferred process for producing a compound of formula (II) comprises the reduction of a compound of formula (IIIA), especially using sodium borohydride in methanol or ethanol at ambient temperature.

A particularly preferred process for producing a compound of formula (II) in which $R^3$ is hydrogen, comprises the reduction of a compound of formula (II) in which $R^3$ is benzyl. This reduction is conveniently carried out by catalytic hydrogenation in ethanol, using palladium on charcoal. A compound of formula (II) in which $R^3$ is hydrogen may also be produced by carrying out an ether cleavage with for example hydrobromic acid on a compound of formula (II) in which $R^3$ is lower alkyl.

The present invention also provides a further process for producing a compound of formula (II) or a salt thereof, which process comprises reacting a compound of formula (IV):

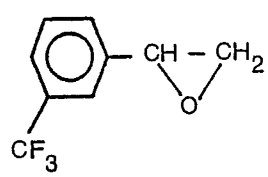

(IV)

with a compound of formula (V):

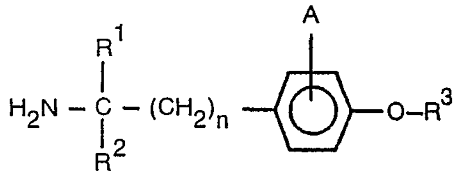

(V)

wherein $R^1$, $R^2$, $R^3$, A and n are as defined in relation to formula (II), and optionally thereafter forming a salt of the compound of formula (II) so formed.

This reaction is conveniently effected in a solvent such as alower alkanol, preferably ethanol.

An additional process for preparing a compound of formula (II) or a salt thereof comprises reacting a compound of formula (IVA):

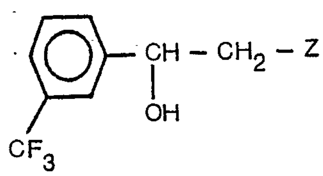

(IVA)

in which Z is a leaving group, such as a tosylate or mesylate group, with a compound of formula (V) as defined above, or, alternatively, reacting a compound of formula (IVB):

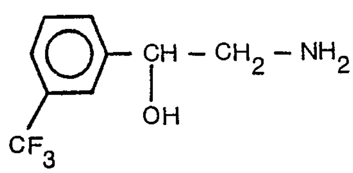

(IVB)

with a compound of formula (IVC):

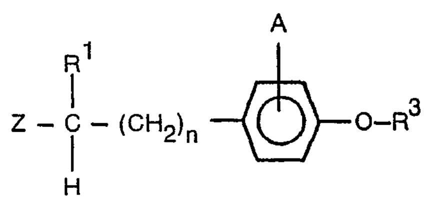

(IVC)

in which $R^1$, $R^3$, A and n are as defined in formula (II) and Z is as defined in formula (IVA).

Compounds of formula (II) and salts thereof, produced by the above processes, may be recovered by conventional methods.

Compounds of formula (III) may themselves be producd by reacting a compound of formula (V):

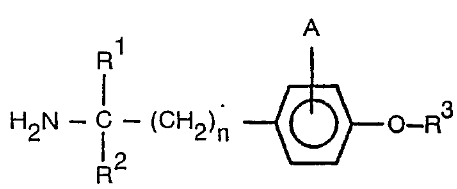

(V)

wherein $R^1$, $R^2$, $R^3$, A and n are as defined in relation to formula (II)
with a compound of formula (VII):

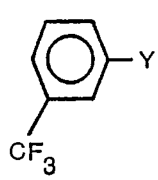

(VII)

wherein:

Y is a moiety which is capable of reacting with the amine of formula (V) thus forming a compound of formula (III). Typical examples of compounds of formula (VII) are:

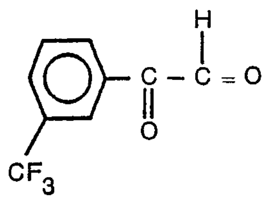

(VIIA)

its hydrate or hemi-acetal of a lower alkanol;

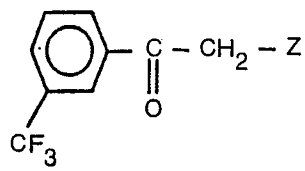

(VIIB)

wherein Z is a halogen atom, preferably bromine; and

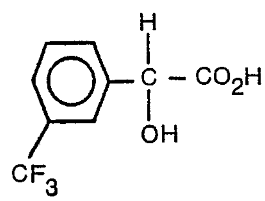

(VIIC)

Coventional conditions suitable for use with the particular compound of formula (VII) may be used for this reaction. Thus the reaction of compound of formula (VIIA) with a compound of formula (V) is conveniently conducted at elevated temperature under conditions resulting in the removal of the water formed during the reaction. A particularly suitable method is to perform the reaction in a solvent, such as benzene, under reflux and azeotropically to remove the water using a Dean and Stark trap.

The reaction of a compound of formula (VIIB) with a compound of formula (V) is conveniently conducted in a polar organic solvent such as acetonitrile or butanone, at an elevated temperature, for instance under reflux.

The reaction of a compound of formula (VIIC) with a compound of formula (V) is conveniently conducted under standard peptide formation reaction conditions.

A particularly preferred process for preparing certain compounds of formula (III) comprises reacting a compound of formula (VIII):

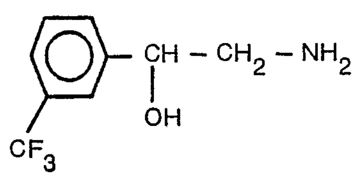

(VIII)

with a compound of the formula (IX):

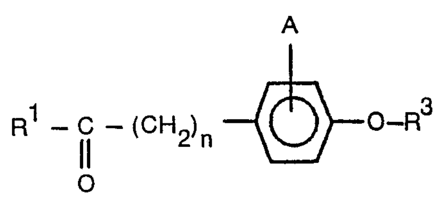

(IX)

wherein $R^1$, $R^3$, A and n are as defined in relation to formula (II).

This reaction is conveniently effected under conditions which result in the removal of water formed during the reaction. A particularly suitable method is to perform the reaction in a solvent, such as benzene

or toluene, under reflux and azeotropically to remove the water using a Dean and Stark trap.

It is often convenient to prepare the compound of formula (III) and reduce it to the desired compound of formula (II) without isolation of the compound of formula (III).

Compounds of formula (II) having a single asymmetric carbon atom may, if desired, be separated into individual enantiomers by conventional means, for example by the use of an optically active acid as a resolving agent. Those compounds of formula (II) having two asymmetric carbon atoms may be separated into diastereoisomeric pairs of enantiomers by, for example, fractional crystallisation from a suitable solvent such as ethyl acetate. The pair of enantiomers thus obtained may be separated into individual stereoisomers by conventional means such as by the use of an optically active acid as a resolving agent.

Suitable optically active acids which may be used as resolving agents are described in "Topics in Stereochemistry" Vol 6, Wiley Interscience, 1971, Allinger, N L and Eliel, W D Eds.

Alternatively any enantiomer of a compound of formula (II) may be obtaioned by stereospecific synthesis using optically pure starting materials of known configuration.

By using single enantiomers of a compound of formula (V) and of a compound of formula (VIIC) a stereospecific synthesis of a compound of formula (III) is achieved. This may then be reduced to a compound of formula (II) without altering the configuration of the two asymmetric carbon atoms. By using single enantiomers of a compound of formula (V) and of a compound of formula (IV) a stereospecific synthesis of a compound of formula (II) is achieved directly. Thus, for example, a compound of formula (V) with the R absolute configuration and a compound of formula (IV) with the R absolute configuration would afford a compound of formula (II) with the RR absolute configuration.

In a modification of the above process for producing compounds of formula (III), the N-benzyl derivative of a compound of formula (V) may be reacted with a compound of formula (VIIB). In this case the N-benzyl derivative of formula (III) is produced and this may be reduced into a compound of formula (II) using a catalytic hydrogenation reaction, especially using palladium on charcoal as catalyst.

A compound of formula (II) or a pharmaceutically acceptable salt thereof (hereinafter "the drug") may be administered as the pure drug, however, it is preferred that the drug be administered as a pharmaceutical composition also comprising a pharmaceutically acceptable carrier.

Accordingly, the present invention also provides a pharmaceutical composition comprising a compound of formula (II) or a pharmaceutically acceptable salt thereof with a pharmaceutically acceptable carrier therefor.

As used herein the terms "pharmaceutical composition" and "pharmaceutically acceptable" embrace compositions and ingredients for both human and veterinary use.

Usually the compositions of the present invention will be adapted for oral administration although compositions for administration by other routes, such as by injection are also envisaged.

Particularly suitable compositions for oral administration are unit dosage forms such as tablets and capsules. Other fixed-unit dosage forms, such as powders presented in sachets, may also be used.

In accordance with conventional pharmaceutical practice the carrier may comprise a diluent, binder, filler, disintegrant, wetting agent, lubricant, colourant, flavourant, or the like.

Typical carriers may, therefore, comprise such agents as microcrystalline cellulose, starch, sodium starch glycollate, polyvinylpyrrolidone, polyvinylpolypyrrolidone, magnesium stearate, sodium lauryl sulphate, sucrose and the like.

Most suitably the composition will be provided in unit dose form. Such unit doses will normally comprise 0.1 to 500 mg of the drug, more usually 0.1 to 250 mg and favourably 0.1 to 100 mg.

The present invention further provides a method for treating hyperglycaemia in humans or animals which method comprises administering an effective, non-toxic amount of a compound of formula (II) or a pharmaceutically acceptable salt thereof to hyperglycaemic humans or animals.

The present invention also provides a method for treating obesity in humans or animals, which method comprises admininstering an effective, non-toxic amount of a compound of formula (II) or a pharmaceutically acceptable salt thereof to obese humans or animals.

The present invention also provides a method for treating inflammation in humans, which comprises topically administering an effective, non-toxic, amount of a compound of formula (II) or a pharmaceutically acceptable salt thereof to humans suffering from inflammation.

The present invention also provides a method for inhibiting platelet aggregation in humans, which comprises administering to the sufferer an effective, non-toxic, amount of a compound of formula (II) or a pharmaceutically acceptable salt thereof.

Conveniently, the drug may be administered as a pharmaceutical composition as hereinbefore defined, and this forms a particular aspect of the present invention.

In treating hyperglycaemic or obese humans the drug may be taken in doses, such a those described above, one to six times a day in a manner such that the total daily dose for a 70 kg adut will generally be about 0.1 to 1000 mg, and more usually about 1 to 500 mg.

In treating hyperglycaemic or obese animals, especially dogs, the drug may be administered by mouth, usually once or twice a day and at about 0.025 mg/kg to 10 mg/kg, for example 0.1 mg/kg to 2 mg/kg.

The invention will now be illustrated with reference to the following Examples.

Example 1

N-[2-(4-Benzyloxyphenyl)-1-methylethyl]-2-hydroxy-2-(3-trifluoromethylphenyl)ethanamine

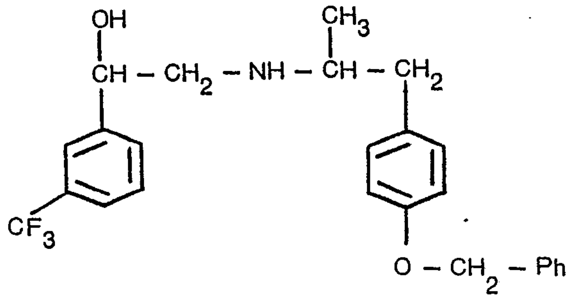

1-(4-Benzyloxyphenyl)propan-2-one (2.65 g) was added to a solution of 2-hydroxy-2-(3-trifluoromethylphenyl)ethanamine (2.05 g) in dry toluene (100 ml) and the solution was boiled under reflux for 2 hours in an apparatus incorporating a water-trap. The solution was cooled and the solvent removed under reduced pressure. The residue dissolved in methanol (100 ml), was cooled to less than 10°C and treated portionwise with sodium borohydride (2.4 g) over 30 minutes. The mixture was stirred for 1 hour at room temperature and the solvent removed under reduced pressure. The residue was dissolved in ethyl acetate, washed with water, dried (magnesium sulphate), filtered and evaporated. Chromatography of the residue on silica gel in 1% methanol-dichloromethane gave 2.5 g of the title compound. mp 84—85°C, as a 50:50 mixture of diastereoisomers.

$^1$Hnmr: τ ($CDCl_3$)

8.9 (3H, d, J = 6Hz), 6.8—7,8 (5H, m) 7.5 (2H, broad, disappears with $D_2O$), 5.3 (1H, m J = 6Hz), 5.0 (2H, s), 3.2 (2H, dd, J = 8Hz), 2.9 (2H, dd, J = 8Hz, J = 2Hz), 2.2—2.7 (9H, m).

Example 2

N-[2-4-Hydroxyphenyl)-1-methylethyl]-2-hydroxy-2-(3-trifluoromethylphenyl)ethanamine

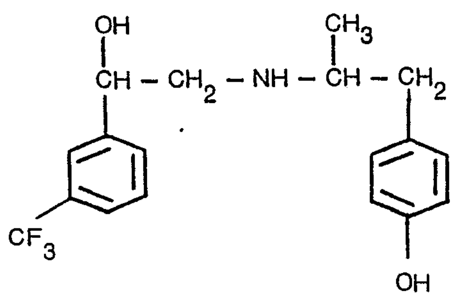

N-[2-(4-Benzyloxyphenyl)-1-methylethyl]-2-hydroxy-2-(3-trifluoromethylphenyl)ethanamine (1.8 g) in ethanol (100 ml) was hydrogenated at ambient temperature and atmospheric pressure in the presence of palladium on charcoal (10%, 0.15 g). When hydrogen uptake ceased the solution was filtered through diatomaceous earth, evaporated and the residue chromatographed on silica-gel in 3% methanoldichloromethane. The title compound (1.0 g) was obtained pure by crystallisation from 50% ethyl acetate-hexane. mp 114—115°C, as a 84:16 mixture of diastereoisomers.

$^1$Hnmr: τ ($CDCl_3$)

8.85 (3H, d,), 6.9—7.6 (5H, d), 6.15 (3H broad, disappears with $D_2O$), 5.4 (1H, m, J = 4Hz), 3.2 (2H, d, J = 6Hz), 3.0 (2H, d, J = 6Hz), 2.3—2.6 (4H, m).

Example 3

N-[2-(3,4-Dimethoxyphenyl)-1-methylethyl]-2-hydroxy-2-(3-trifluoromethylphenyl)ethanamine

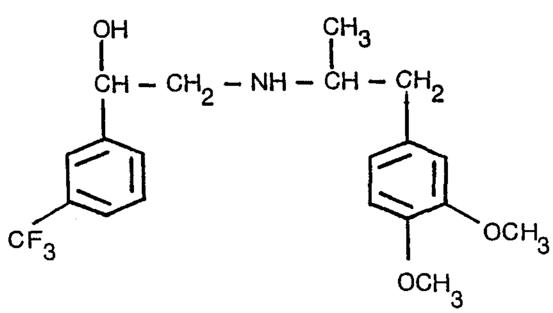

This compound was prepared from 1-(3,4-dimethoxyphenyl)propan-2-one and 2-hydroxy-2-(3-trifluoromethylphenyl)ethanamine by an analogous procedure to that described in Example 1. Purification by chromatography on silica gel in 3% methanol-chloroform gave analytically pure product, mp 69.5—71°C, as a 50:50 mixture of diastereoisomers.

$^1$Hnmr: τ ($CDCl_3$)

8.9 (3H, d), 6.7—7.8 (7H, m; 2H disappear with $D_2O$), 6.2 (6H, s), 5.3 (1H, m), 3.1—3.5 (3H, m), 2.2—2.7 (4H, m).

Example 4

N-[2-(3-Fluoro-4-methoxyphenyl)-1-methylethyl]-2-hydroxy-2-(3-trifluoromethylphenyl)ethanamine.

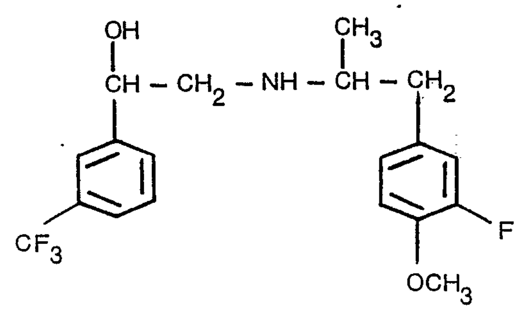

Starting from 1-(3-fluoro-4-methoxyphenyl)propan-2-one (2.73 g) and 2-hydroxy-2-(3-trifluoromethylphenyl)ethanamine (3.07 g), N-[2-(3-fluoro-4-methoxyphenyl)-1-methylethyl]-2-hydroxy-2-(3-trifluoromethylphenyl)ethanamine was prepared by a procedure analogous to that described in Example 1. Purification by chromatography on silica gel in 5% methanol in dichloromethane gave the product, mp 72—4°C, (ether-hexane) as a 17:83 mixture of diastereoisomers.

$^1$Hnmr: τ ($CDCl_3$)

8.95 (3H, d), 6.9—7.6 (7H, complex; 2H exchange with $D_2O$), 6.15 (3H, s), 5.35 (1H, m), 2.95—3.3 (3H, complex), 2.3—2.6 (4H, complex).

Example 5

N-[2-(4-Methoxyphenyl)-1-methylethyl]-2-hydroxy-2-(3-trifluoromethylphenyl)ethanamine.

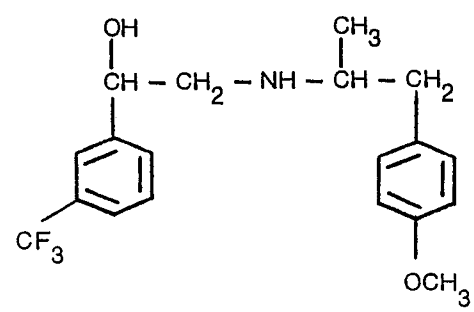

A mixture of 1-(4-methoxyphenyl)propan-2-one (0.9 g) and 2-hydroxy-2-(3-trifluoromethylphenyl)ethanamine (1.03 g) in dry benzene (50 ml) was heated under reflux using a Dean and Stark head for two hours and evaporated to dryness. The residue was dissolved in methanol and hydrogenated at NTP using platinum oxide (30 mg) as catalyst. After filtration and evaporation the residue was crystallised from ethylacetate-hexane to give N-[2-(4-methoxyphenyl)-1-methylethyl]-2-hydroxy-2-(3-trifluoromethylphenyl)ethanamine, mp 75—8°C, as a 61:39 mixture of diastereoisomers.

$^1$Hnmr: τ ($CDCl_3$)

8.95 (3H, d), 6.9—7.65 (7H, complex; 2H exchange with $D_2O$), 6.25 (3H, s), 5.4 (1H, m), 3.05—3.3 (2H, dd), 2.8—3.0 (2H, dd), 2.3—2.6 (4H, complex).

Example 6

N-[2-(4-Benzyloxy-3-methoxyphenyl)-1-methylethyl]-2-hydroxy-2-(3-trifluoromethylphenyl)ethanamine.

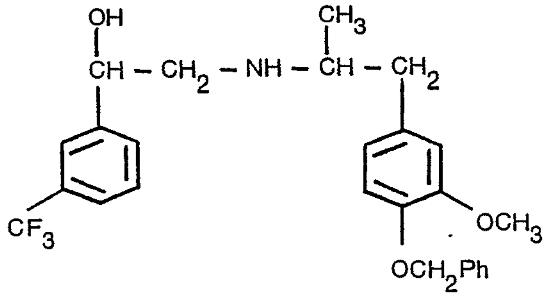

Starting from 1-(4-benzyloxy-3-methoxyphenyl)propan-2-one (2.5 g) and 2-hydroxy-2-(3-trifluoromethylphenyl)ethanamine (2.1 g), N-[2-(4-benzyloxy-3-methoxyphenyl)-2-methylethyl]-2-hydroxy-2-(3-trifluoromethylphenyl)ethanamine was prepared by a procedure analogous to that described in Example 1. Crystallisation from ethyl acetate gave the product, mp 92—6°C, as a 71:29 mixture of diastereoisomers.

$^{1}$Hnmr: τ ($CDCl_3$)

8.95 (3H, d), 6.85—7.7 (7H, complex; 2H exchange with $D_2O$), 6.15 (3H, s), 5.4 (1H, m), 4.9 (2H, s), 3.05—3.5 (3H, m), 2.—2.8 (9H, complex).

Example 7

N-[2-(4-Benzyloxy-3-chlorophenyl)-1-methylethyl]-2-hydroxy-2-(3-trifluoromethylphenyl)ethanamine

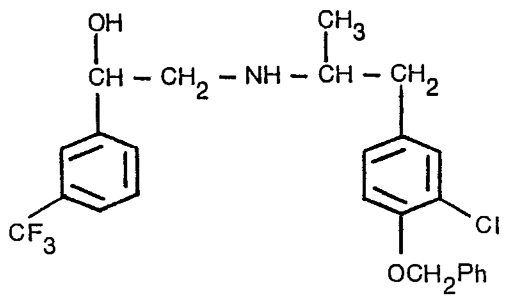

Starting from 2-hydroxy-2-(3-trifluoromethylphenyl)ethanamine (4.01 g) and 1-(4-benzyloxy-3-chlorophenyl)propan-2-one (5.36 g), N-[2-(4-benzyloxy-3-chlorophenyl)-1-methylethyl]-2-hydroxy-2-(3-trifluoromethylphenyl)ethanamine was prepared by a procedure analogous to that described in Example 1. Purification by chromatography on silica gel in 3 to 5% methanol in dichloromethane gave the product, mp 92—105°C, as a 51:49 mixture of diastereoisomers.

$^{1}$Hnmr: τ ($CDCl_3$)

8.95 (3H, d), 6.8—7.8 (7H, m), 5.4 (1H, m), 4.9 (2H, s), 2.2—3.2 (12H, complex).

Example 8

N-[2-(3-Chloro-4-hydroxyphenyl)-1-methylethyl]-2-hydroxy-2-(3-trifluoromethylphenyl)ethanamine

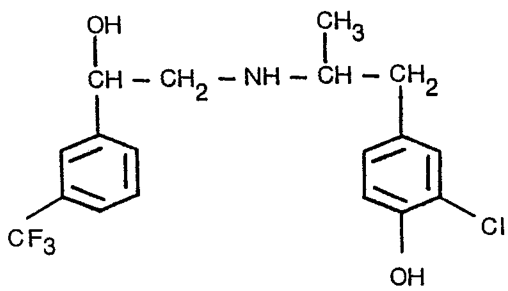

Starting from 1-(3-chloro-4-hydroxy-phenyl)propan-2-one (2.38 g) and 2-hydroxy-2-(3-trifluoromethyl phenyl)ethanamine (2.65 g), N-[2-(3-chloro-4-hydroxyphenyl)-1-methylethyl]-2-hydroxy-2-(3-trifluoromethylphenyl)ethanamine was prepared by a procedure analogous to that described in Example 1. Purification by column chromatography on silica gel in 4% methanol in dichloromethane gave the product, mp 48—62°C, as a 52:48 mixture of diastereoisomers.

$^{1}$Hnmr: τ ($CDCl_3$)

8.9 (3H, d), 6.9—7.6 (5H, complex), 6.45 (3H), 5.4 (1H, m), 2.8—3.2 (3H, complex), 2.35—2.7 (4H, complex).

Example 9

N-[2-(3-Fluoro-4-methoxyphenyl)-1-methylethyl]-2-hydroxy-2-(3-trifluoromethylphenyl)ethanamine hydrobromide-(RR,SS) diastereoisomer

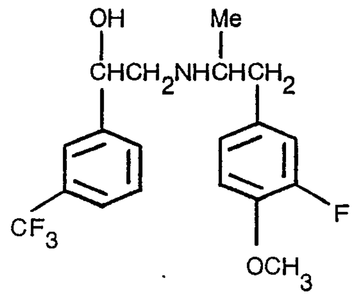

HBr. (RR, SS) diastereoisomer

Hydrogen bromide was passed through a solution of N-[2-(3-fluoro-4-methoxyphenyl)-1-methylethyl]-2-hydroxy-2-(3-trifluoromethylphenyl)ethanamine in diethyl ether until precipitation was complete. The solvent was removed under vacuum and the residual solid recrystallised from ethyl acetate to give the title compound, m.p. 160—70°, of analytical purity and 92% diastereisomeric purity.

Example 10

N-[2-(4-$^n$Decyloxyphenyl)-1-methylethyl]-2-hydroxy-2-(3-trifluoromethylphenyl) ethanamine

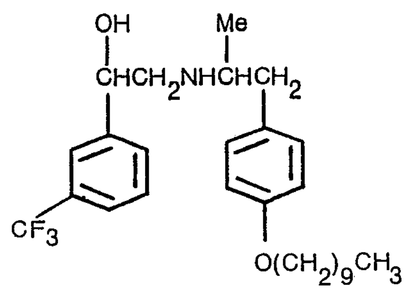

The title compound, m.p. 41—44°, was prepared as a 49:51 mixture of diastereoisomers, from 1-(4-$^n$decyloxyphenyl)propan-2-one and 2-hydroxy-2-(3-trifluoromethylphenyl) ethanamine by an analogous procedure to that described in Example 1.

$^1$H nmr

τ ($CDCl_3$). 8.95—9.2 (3H, m), 8.9 (3H, d), 8.0—8.8 (16H, complex) 6.9—7.6 (7H, complex; 2H exchange with $D_2O$), 6.05 (2H, t), 5.3 (1H, m), 2.8—3.25 (4H, complex), 2.2—2.55 (4H, complex).

Example 11

N-[2-(4-$^n$Pentyloxyphenyl)-1-methylethyl]-2-hydroxy-2-(3-trifluoromethylphenyl) ethanamine

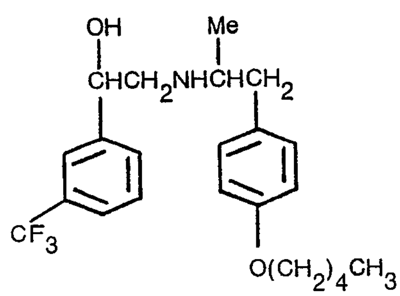

The title compound, m.p. 59—63° (hexane), was prepared, as a 50:50 mixtue of diastereoisomers, from 1-(4-$^n$pentyloxyphenyl)propan-2-one and 2-hydroxy-2-(3-trifluoromethylphenyl) ethanamine by an analogous procedure to that described in Example 1.

$^1$H nmr

τ ($CDCl_3$). 8.85—9.2 (6H, d on m), 8.0—8.8 (6H, complex), 6.85—7.7 (7H, complex; 2H exchange with $D_2O$), 6.1 (2H, t), 5.35 (1H, m), 2.85—3.3 (4H, complex), 2.2—2.65 (4H, complex).

Example 12

N-[2-(4-Isopropoxyphenyl)-1-methylethyl]-2-hydroxy-2-(3-trifluoromethylphenyl) ethanamine

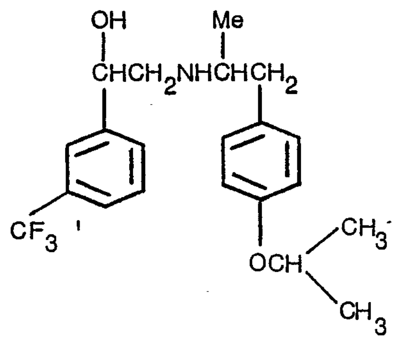

The title compound, m.p. 79—83° (hexane), was prepared, as a 72:28 mixture of diastereoisomers, from 1-(4-isopropoxyphenyl)-propan-2-one and 2-hydroxy-2-(3-trifluoromethylphenyl) ethanamine by an analogous procedure to that described in Example 1.

'H nmr

τ ($CDCl_3$) 8.95 (3H, d), 8.7 (6H, d), 6.9—7.65 (7H, complex; 2H exchange with $D_2O$), 5.2—5.7 (2H, complex), 2.8—3.3 (4H, complex), 2.25—2.6 (4H, complex).

Example 13

N-[2-(4-Methoxyphenyl)ethyl-2-hydroxy-2-(3-trifluoromethylphenyl) ethanamine

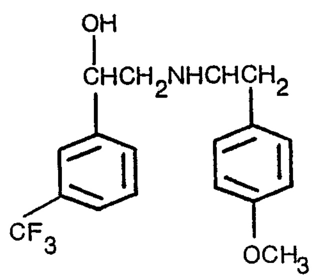

A solution of N-[2-(4-methoxyphenyl)ethyl]-2-hydroxy-1-oxo-2-(3-trifluoromethylphenyl) ethanamine (2.39g) in dry tetrahydrofuran (15ml) was added slowly to a refluxing suspension of lithium aluminium hydride (0.65g) in tetrahydrofuran (50ml) and then heated under reflux for 24 hours. After careful addition of water (0.65ml), 10% aqueous sodium hydroxide (0.65ml) and water (1.3ml), filtration and evaporation of the filtrate gave the crude product. Chromatography on silica gel using 1% methanol in dichloromethane gave the title compound, m.p. 85—7°, of analytical purity.

'H nmr

τ ($CDCl_3$). 6.9—7.6 (8H, complex); 6.2 (3H, s); 5.3 (1H, dd), 2.7—3.3 (4H, dd), 2.2—2.6 (4H, complex).

Example 14

N-[3-(4-Methyloxyphenyl)-1,1-dimethylpropyl]-2-hydroxy-2-(3-trifluoromethylphenyl)ethanamine

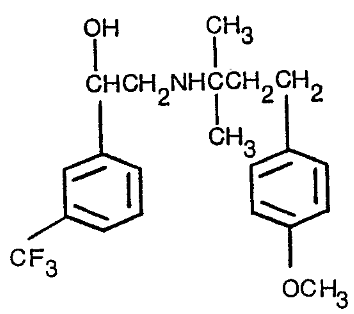

3-Trifluoromethylmandelic acid (2.14 g) was coupled with 3-(4-methoxyphenyl)-1,1-dimethylpropylamine (1.91 g) by an analogous procedure to that described in Description 8. The crude amide so obtained was reduced with lithium aluminium hydride in tetrahydrofuran by an analogous procedure to that described in Example 13. Chromatography on silica gel using 1% methanol in

dichloromethane as eluent gave the title compound, mp 50—53°C (hexane—diethyl ether) of analytical purity.

$^1H$ nmr

τ ($CDCl_3$) 8.85 (6H, s), 8.2—8.55 (2H, m), 7.0—7.65 (6H, m; 2H exchanges with $D_2O$), 6.25 (3H, s), 5.4 (1H, dd), 2.75—3.3 (4H, dd), 2.2—2.55 (4H, m).

Example 15

N-[2-(4-Methoxyphenyl)-1-methylethyl]-2-hydroxy-2-(3-trifluoromethylphenyl)ethanamine hydrochloride-(R,R) isomer

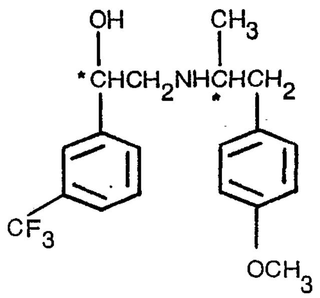

HCl; (R,R) isomer

(R)-3-Trifluoromethylmandelic acid (2.68 g) and (R)-2-(4-methoxyphenyl)-1-methyl ethanamine (2.07 g) were coupled by an analogous procedure to that described in Description 8. The crude amide so obtained was reduced with lithium aluminium hydride in tetrahydrofuran by an analogous procedure to that described in Example 13. The crude product obtained was purified by chromatography on silica gel using 2% methanol in dichloromethane as eluent, converted to its hydrochloride salt by addition of ethereal hydrogen chloride and crystallised from ethyl acetate-ether-hexane to give the title compound, mp 92—4°C $[\alpha]D^{25} -44.31°$ (MeOH) of 97% enantiomeric purity.

$^1H$ nmr

τ (DMSO-$d_6$) 8.9 (3H, d), 7.15—7.4 (1H, m), 6.4—7.0 (4H, m), 6.25 (3H, s), 4.75 (1h, m), 3.5 (1H, exchanges with $D_2O$), 2.6—3.2 (4H, dd), 2.1—2.5 (4H, complex), 0.95 (1H, broad; exchanges with $D_2O$), 0.25 (1H, broad, exchanges with $D_2O$).

Description 1

1-(3-Fluoro-4-methoxyphenyl)-2-nitroprop-1-ene

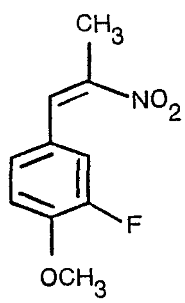

A mixture of 3-fluoro-4-methoxybenzaldehyde (5.0 g) and n-butylamine (5.0 g) in dry benzene (100 ml) was heated under reflux using a Dean and Stark head until the required amount of water had been collected, cooled and solvent removed under vacuum. The residue was dissolved in acetic acid (30 ml), nitroethane (4.0 g) added and the mixture stirred at 100°C for two hours, cooled, poured into water and filtered to give the title compound.

$^1H$nmr: τ ($CDCl_3$)

7.5 (3H, s), 6.05 (3H, s), 2.6—3.2 (3H, complex), 2.0 (1H, S).

Description 2

1-[3-fluoro-4-methoxyphenyl)propan-2-one

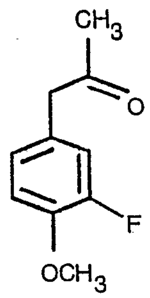

Concentrated hydrochloric acid (30 ml) was added dropwise, over 45 minutes, to a stirred mixture of iron powder (6 g; and 1-(3-fluoro-4-methoxyphenyl)-2-nitroprop-1-ene (5.7 g) in tetrahydrofuran (100 ml) at reflux. The mixture was heated under reflux for a further two hours, cooled, filtered and evaporated. Water (100 ml) was added to the residue and extracted with dichloromethane. The dried (magnesium sulphate) extracts were evaporated to dryness to give the title compound as an oil.

Description 3

1-(4-Benzyloxy-3-methoxyphenyl)-2-nitroprop-1-ene

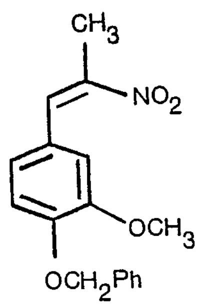

The title compound, MP 78—80°C, was prepared from 4-benzyloxy-3-methoxybenzaldehyde (24.2 g), n-butylamine (20 ml) and nitroethane (20 ml), by an analogous procedure to that descried in Description 1.

$^{1}$Hnmr: τ ($CDCl_3$)

7.5 (3H, s), 6.1 (3H, s), 4.8 (2H, s), 3.05 (3H, s), 2.45—2.8 (5H, m), 2.0 (1H, s).

Description 4

1-(4-Benzyloxy-3-methoxyphenyl)propan-2-one

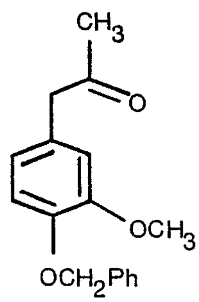

The title compound was prepared from 1-(4-benzyloxy-3-methoxy-phenyl)-2-nitroprop-1-ene by an analogous procedure to that described in Description 2.

Description 5

1-(4-Benzyloxy-3-chlorophenyl)-2-nitroprop-1-ene

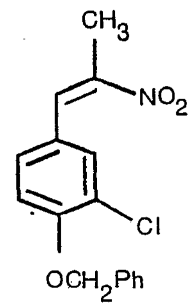

The title compound was prepared from 4-benzyloxy-3-chlorobenzaldehyde (15.14 g), n-butylamine (9 ml) and nitroethane (11 ml) by an analogous procedure to that described in Description 1.

$^1$Hnmr: τ ($CDCl_3$)

7.55 (3H, s), 4.8 (2H, s), 2.4—3.05 (8H, complex), 2.0 (1H, s).

Description 6

1-(4-Benzyloxy-3-chlorophenyl)propan-2-one

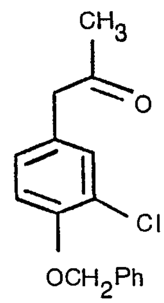

The title compound was prepared from 1-(4-benzyloxy-3-chlorophenyl)-2-nitroprop-1-ene by an analogous procedure to that described in Description 2. The product was recrystallised from diethylether-hexane.

$^1$Hnmr: τ ($CDCl_3$)

7.9 (3H, s), 6.4 (2H, s), 4.9 (2H, s), 2.4—3.2 (8H, complex).

Description 7

1-(3-Chloro-4-hydroxyphenyl)propan-2-one

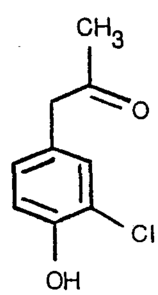

The title compound was prepared from 1-(4-benzyloxy-3-chlorophenyl)propan-2-one by hydrogenation in ethyl acetate using 10% palladium on charcoal as catalyst.

Description 8

N-[2-(4-Methoxyphenyl)ethyl]-2-hydroxy-1-oxo-2-(3-trifluoromethylphenyl)ethanamine

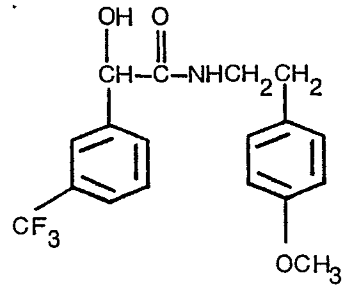

Dicyclohexylcarbodiimide (2.02g) in DMF (8ml) was added to a solution of 1-hydroxybenzotriazole (1.37g), 2-(4-methoxyphenyl) ethanamine (1.51g) and 3-trifluoromethyl mandelic acid (2.14g) in DMF (40ml) and the mixture stirred for 14 hours, filtered then evaporated to dryness. The residue was dissolved in ethyl acetate, washed with aq. 4M HCl and sodium bicarbonate solution, dried ($MgSO_4$), filtered and evaporated to dryness to give the title compound, as an oil, which was used without further purification.

'H nmr

τ ($CDCl_3$) 7.35 (2H, t), 6.6 (2H, m), 6.25 (3, s), 5.0 (1H, broadened s), 2.9—3.35 (4H, dd), 2.2—2.6 (4H, complex).

Description 9

4-Isopropoxybenzaldehyde

A mixture of 4-hydroxybenzaldehyde (20g), anhydrous potassium carbonate (25g), potassium iodide (0.5g) and 2-iodopropane (27g) in 2-butanone (300ml) was heated under reflux, with stirring, overnight, cooled, filtered and evaporated to dryness. The residue was dissolved diethyl ether, washed with aqueous sodium hydroxide and brine, dried ($MgSO_4$), filtered and evaporated to dryness. Distillation of the residue gave the title compound, bp 108—112°/3mm as an oil.

'H nmr

τ ($CDCl_3$) 8.65 (6H, d), 5.35 (1H, m), 3.1 (2H, d), 2.2 (2H, d), 0.15 (1H, s).

Description 10

4-$^n$Pentyloxybenzaldehyde

The title compound, bp 148—152°/2mm, was prepared from 1-bromopentane and 4-hydroxybenzaldehyde by an analogous procedure to that described in Description 9.

'H nmr

τ ($CDCl_3$). 8.0—9.3 (9 H, complex), 6.05 (2H, t), 3.05 (2H, d), 2.2 (2H, d), 0.2 (1H, s).

Description 11

4-$^n$Decyloxybenzaldehyde

4-Hydroxybenzaldehyde (12.2g) was added to a solution of sodium ethoxide (prepared by addition of sodium (2.5g) to ethanol (100ml) in ethanol and stirred for 15 minutes. $^n$Decylbromide (22.0g) in ethanol (50ml) was added and the mixture refluxed for one day, cooled, filtered and evaporated to dryness. The residue was dissolved in diethyl ether, washed with aqueous sodium hydroxide, dried ($MgSO_4$), filtered and evaporated to dryness. Distillation of the residue gave the title compound, bp 206—8°/4mm.

'H nmr

τ ($CDCl_3$). 7.95—9.4 (19H, complex), 6.0 (2H, t), 3.0 (2H, d), 2.20 (2H, d), 0.1 (1H, s).

Description 12

1-(4-Isopropoxyphenyl)-2-nitroprop-1-ene

A mixture of 4-isopropoxybenzaldehyde (17.0g) and n-butylamine (10 g) in toluene was heated under reflux using a Dean and Stark head for two hours, cooled and evaporated to dryness. The residue was dissolved in acetic acid (100ml), nitroethane (14ml) added and the mixture heated at 100° for 2 hours then poured into water. The crude product was obtained by extraction with diethylether, drying over $MgSO_4$, filtering and evaporation of solvent, and used without further purification.

'H nmr

τ ($CDCl_3$) 8.6 (6H, d), 7.55 (3H, s), 5.4 (1H, m), 3.1 (2h, d), 2.6 (2H, d), 2.0 (1H, s).

Description 13

1-(4-$^n$Pentyloxyphenyl)-2-nitroprop-1-ene

The title compound was prepared, as an oil, from 4-$^n$pentyloxybenzaldehyde by an analogous procedure to that described in Description 12 and used without further purification.

'H nmr

τ ($CDCl_3$), 8.0—9.3 (9H, complex) 7.5 (3H, s), 5.95 (2H, t), 3.0 (2H, d), 2.5 (2H, d), 1.95 (1H, s).

Description 14

1-(4-$^n$Decyloxyphenyl)-2-nitroprop-1-ene

The title compound was prepared, as a solid, from 4-$^n$decyloxybenzaldehyde by a analogous procedure to that described in Description 12, and subsequently used without further purification.

'H nmr

τ ($CDCl_3$). 8.0—9.3 (19H, complex), 7.5 (3H, s), 6.0 (2H, t), 3.05 (2H, d), 2.55 (2H, d), 1.95 (1H, s).

Description 15

1-4-Isopropoxyphenyl)propan-2-one

Concentrated hydrochloric acid (90ml) was added dropwise over 45 minutes, to a stirred mixture of iron powder (17.5g) and 1-(4-isopropoxyphenyl)-2-nitroprop-1-ene (16.0g) in tetrahydrofuran (50ml) at reflux. The mixture was heated undeer reflux for a further hour, cooled, filtered through celite and the THF removed under vacuum. The residue was diluted with water, extracted with diethyl ether, washed with brine, dried ($MgSO_4$), filtered and evaporated to dryness. Distillation of the residue gave the title compound, bp 120—6°/1.5mm.

'H nmr

τ ($CDCl_3$). 8.7 (6H, d), 7.8 (3H, d), 6.4 (2H, s), 5.5 (1H, m), 2.7—4.4 (4H, dd).

Description 16

1(4-$^n$Pentyloxyphenyl)propan-2-one

The title compound, bp 140—150°/1.5mm, was prepared from 1-(4-$^n$pentyloxyphenyl)-2-nitroprop-1-ene, by an analogous procedure to that described in Description 15.

'H nmr

τ ($CDCl_3$) 8.05—9.3 (9H, complex), 7.9 (3H, s), 6.4 (2H, s), 6.1 (2H, t), 2.7—3.3 (4H, dd).

Description 17

1-(4-$^n$Decyloxyphenyl)propan-2-one

The title compound, bp 190—5°/1mm, was prepared from 1-(4$^n$decyloxyphenyl)-2-nitroprop-1-ene by an analogous procedure to that described in Description 15.

'H nmr

τ ($CDCl_3$). 8.0—9.3 (19H, complex), 7.85 (3H, s), 6.4 (2H, s), 6.05 (2H, t), 2.8—3.4 (4H, dd).

Description 18

N-[1-(R)-1-Phenylethyl]-1-(R)-2-(4-methoxyphenyl)-1-methyl ethanamine hydrochloride

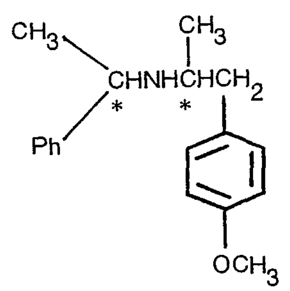

HCl (R,R isomer)

A mixture of d-(+)-α-methylbenzylamine (11.07 g) and 1-(4-methoxyphenyl)propan-2-one (15.0 g) in dry benzene (200 ml) was heated under reflux using a Dean and Stark apparatus until the theoretical amount of water had been collected. The solvent was evaporated and replaced by methanol. Raney nickel was added and the mixture hydrogenated at atmospheric pressure for one day. The catalyst was filtered off, the solvent evaporated and the residue distilled to give the title compound as its free base, bp 162—7°C/1.5 mm, $[\alpha]^D_{25}$+35.3° (MeOH).

$^1$H nmr

τ ($CDCl_3$) 9.1 (3H, d), 8.7 (4H, d on m), 7.0—7.65 (3H, m), 5.8—6.25 (4H, s on q), 2.8—3.3 (4H, dd), 2.5—2.8 (5H, m).

The hydrochloride salt, mp 182—5°C, $[\alpha]^D_{25}$+33.3° (MeOH) was obtained by addition of ethereal hydrogen chloride to an ethereal solution of the free base and subsequent filtration.

Description 19
(R)-2-(4-Methoxyphenyl)-1-methylethanamine hydrochloride

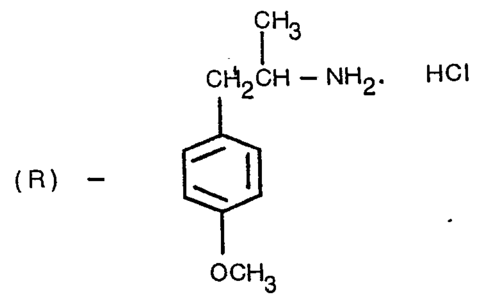

N-[1-(R)-1-Phenylethyl]-1-(R)-2-(4-methoxyphenyl)-1-methylethanamine hydrochloride (10.0 g) in methanol was hydrogenated over 5% Pd/C at 120 psi at 60°/c for 3 days, with mechanical stirring. The catalyst was removed by filtration, the solvent evaporated and the residual solid recrystallised from methanol-diethyl ether to give the title compound, mp 249—51°, $[\alpha]^{D}_{25}$−9.30° (MeOH) of analytical purity.
$^1$H nmr
τ (DMSO-$d_6$) 8.9 (3H, d), 6.45—7.4 (3H, complex), 6.25 (3H, s), 2.65—3.15 (4H, dd), 1.2—2.2 (3H, broad; exchange with $D_2O$).

Demonstration of Effectiveness of Compounds

(I) *Hypoglycaemic activity*

Female CFLP mice, each weighing approximately 25 g, were fasted for 24 hours prior to the study. The compounds under study were administered orally as an aqueous solution to each of 7 mice. 7 mice were given water as a control. 30 minutes later a blood sample (20 µl) was obtained from the tail for the analyst of blood glucose. Immediately after taking this blood sample, glucose (1 g/kg body weight) was administered subcutaneously to each mouse. Blood samples were then obtained from each mouse at 30 minute intervals for 120 minutes.

Compounds that produced a significant ($P<0.05$) reduction of blood glucose, compared with control mice given water, at any time interval, were considered active. The area under the blood glucose curve over the 2 hour period after the administration of the glucose was calculated for each compound and compared with the value for control animals.

| Compound of Example No. | Dose ($\mu$ moles/kg po) | Reduction in area under Blood glucose curve (%) |
|---|---|---|
| Control | – | 0 |
| 1 | 12.5 | 36 |
| 2 | 12.5 | 59 |
| 3 | 12.5 | 43 |
| 4 | 1.0 | 28 |
| 5 | 1.0 | 27 |
| 7 | 12.5 | 28 |
| 8 | 2.5 | 11 |
| 9 | 2.5 | 18 |
| 10 | 5.0 | 48 |
| 11 | 2.5 | 47 |
| 12 | 2.5 | 52 |
| 13 | 5.0 | 27 |
| 14 | 100 | 34 |

*Toxicity*

No toxicity was observed during the above experiments.

(II) *Effect on energy expenditure*

The effect of the compounds on the energy expenditure of mice was demonstrated by means of the following procedure.

Female CFLP mice each weighing approximately 24 g, were given food and water *ad lib* before and during the experiment. The compounds were dissolved in water by addition of one mole of hydrochloric acid per mole of compound and these solutions were administered orally to each of 12 mice. A further 12 mice were dosed orally with water. The mice were placed in boxes through which air was drawn and the oxygen content of the air leaving the boxes was measured. The energy expenditure of the mice was calculated for 21 hours after dosing from the volume of air leaving the boxes and its oxygen content following the principles described by J B de v Weir, *J Physiol* (London) *109,* 1—9, (1949). The food intake of the mice was measured over this same period of 21 hours. The results are expressed as a percentage of the mean food intake or rate of energy expenditure of the mice dosed with water.

| Compound of Example No. | Dose (mg/kg po) | Mean Energy Expenditure (%) | Mean Food Intake (%) |
|---|---|---|---|
| Control | – | 100 | 100 |
| 1 | 23.8 | 115 | 100 |
| 2 | 18.8 | 126 | 90 |
| 3 | 21.3 | 109 | 100 |
| 4 | 20.7 | 113 | 86 |
| 5 | 19.6 | 119 | 88 |
| 6 | 25.6 | 114 | 75 |
| 8 | 20.8 | 118 | 88 |
| 9 | 25.5 | 115 | 78 |
| 10 | 26.7 | 118 | 89 |
| 11 | 23.6 | 118 | 106 |
| 12 | 21.2 | 122 | 94 |
| 13 | 18.9 | 112 | 104 |

(III) *Cardiac Activity*

Rat hearts were perfused by the Langendorff procedure as follows:

Hearts were disssected free within 30 seconds of death and reverse perfused via the aorta and coronary vessels with Krebs-Ringer bicarbonate solution (pH 7.4, 37°C) gassed with 95% oxygen:5% carbon dioxide at a flow rate between 8—12 $cm^3$/minute. Responses were observed after injection of drug dissolved in isotonic saline into the perfusion media. Heart rate and tension were displayed on an Ormed MXP2 recorder via a tension transmitter and heart ratemeter.

Results are expressed as a percentage of the maximum response due to salbutamol.

| Compound of Example No. | Dose added to perfusate ($\mu$g) | Heart Tension | Heart Rate |
|---|---|---|---|
| 1 | 10 | 7 | 0 |
| 2 | 10 | 64 | 73 |
| 3 | 10 | 33 | 14 |
| 4 | 10 | 5 | 0 |
| 5 | 10 | 8 | 0 |
| 6 | 10 | 25 | 6 |
| 7 | 10 | 0 | 0 |
| 8 | 10 | 8 | 23 |
| 9 | 10 | 4 | 16 |

(IV) *Anti-inflammatory Activity*

The method used is based on that described by G. Tonelli *et al* (Endocrinology, *77*,, 625—634, 1965). An inflammation is induced in the rat ear by the application of 50 µl of a 1% solution of croton oil in tetrahydrofuran, test compounds being dissolved in the irritant vehicle. After 6 hours the inflammation is

assessed by killing the animals and weighing the ears. Topical anti-inflammatory activity of a compound is generally considered to be shown when a significant (5% level) reduction in ear weight is seen compared to non-drug treated control.

| Compound of Example No. | Dose mg/rat ear | Activity |
|---|---|---|
| 2 | 1.0 | Active |
| 3 | 1.0 | Active |

(V) *Platelet Aggregation Inhibition Activity — In Vitro*

Collagen-induced platelet aggregation in human whole blood *in vitro.*

Collagen added to stirred citrated blood causes platelet aggregation. As the platelets aggregate, the concentration of single platelets falls. Hence the aggregation response can be quantified as a per cent fall in single platelet count. Inhibitors of aggregation, such as aspirin and dipyridamole, reduce the response collagen.

Blood drawn from volunteers who denied taking aspirin within the previous seven days was mixed with 0.1 vol 129 mM trisodium citrate, dispensed into 0.5 ml aliquots and kept at 25°. Collagen (Hormon-Chemie, Munich) or 154 mM NaCl (control) was added to each aliquot of blood stirred at 1100 rpm in an aggregometer at 37° after 3 mins preincubation with compound or appropriate solvent. Aggregates were fixed by addition of 0.5 vol 4.5% formaldehyde or 0.05 vol phosphate-buffered glutaraldehyde. Single platelets were counted electronically. Responses, expressed as per cent fall in platelet count, were obtained to a range of collagen concentrations. The concentration producing a 50% maximal effect (EC50) was estimated from $\log_{10}$ concentration-response curves. Dose-ratios were calculated by dividing the EC50 obtained in the presence of compound under test by the control EC50.

Results are summarised in the table.

| Compound | Final concentration in whole blood ($\mu$M) | Dose-ratio |
|---|---|---|
| Aspirin | 200 | 2.4 ± 0.3(3) |
| Dipyridamole | 200 | 3.7 ± 1.1(4) |
| Example 2 | 300 | 2.2 |
| Example 4 | 300 | 2.3 |

**Claims**

1. A compound of formula (II):

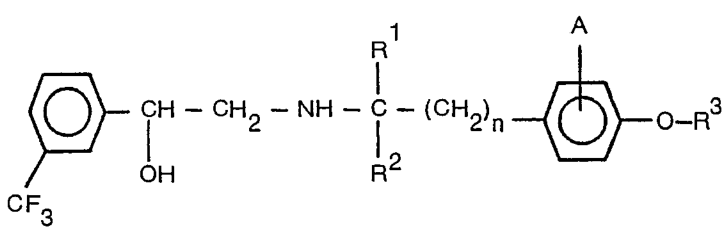

(II)

or a salt thereof;

wherein

$R^1$ is hydrogen or methyl;

$R^2$ is hydrogen or methyl;

$R^3$ is hydrogen, $C_{1-12}$ alkyl, $C_{1-10}$ cycloalkyl, or benzyl optionally substituted with $C_{1-6}$ alkyl, $C_{1-6}$ alkoxy, or halogen,

n is 1 or 2;

and

A is hydrogen, halogen, hydroxyl, $C_{1-6}$ alkyl or $C_{1-6}$ alkoxy.

2. A compound according to claim 1, in which $R^3$ is hydrogen, $C_{1-6}$ alkyl, or benzyl optionally substituted with $C_{1-6}$ alkyl, $C_{1-6}$ alkoxy or halogen.

3. A compound according to claim 1 or claim 2, in which $R^1$ is hydrogen and $R^2$ is methyl.

4. A compound according to claim 1, selected from
N-[2-(4-benzyloxyphenyl)-1-methylethyl]-2-hydroxy-2-(3-trifluoromethylphenyl)ethanamine,
N-[2-(4-hydroxyphenyl)-1-methylethyl]-2-hydroxy-2-(3-trifluoromethylphenyl)ethanamine,
N-[2-(3,4-dimethoxyphenyl)-1-methylethyl]-2-hydroxy-2-(3-trifluoromethylphenyl)ethanamine,
N-[2-(3-fluoro-4-methoxyphenyl)-1-methylethyl]-2-hydroxy-2-(3-trifluoromethylphenyl)ethanamine,
N-[2-(4-methoxyphenyl)-1-methylethyl]-2-hydroxy-2-(3-trifluoromethylphenyl)ethanamine,
N-[2-(4-benzyloxy-3-methoxyphenyl)-1-methylethyl]-2-hydroxy-2-(3-trifluoromethylphenyl)ethanamine,
N-[2-(4-benzyloxy-3-chlorophenyl)-1-methylethyl]-2-hydroxy-2-(3-trifluoromethylphenyl)ethanamine,
N-[2-(3-chloro-4-hydroxyphenyl)-1-methylethyl]-2-hydroxy-2-(3-trifluoromethylphenyl)ethanamine,
N-[2-(3-fluoro-4-methoxyphenyl)-1-methylethyl]-2-hydroxy-2-(3-trifluoromethylphenyl)ethanamine hydrobromide-(RR,SS) diastereoisomer,
N-[2-(4-$^n$Decyloxyphenyl)-1-methylethyl]-2-hydroxy-2-(3-trifluoromethylphenyl)ethanamine,
N-[2-(4-$^n$Pentyloxyphenyl)-1-methylethyl]-2-hydroxy-2-(3-trifluoromethylphenyl)ethanamine,
N-[2-(4-isopropoxyphenyl)-1-methylethyl]-2-hydroxy-2-(3-trifluoromethylphenyl)ethanamine,
N-[2-(4-Methoxyphenyl)ethyl]-2-hydroxy-2-(3-trifluoromethylphenyl)ethanamine,
N-[3-(4-methoxyphenyl)-1,1-dimethylpropyl]-2-hydroxy-2-(3-trifluoromethylphenyl)ethanamine,
or
N-[2-(4-methoxyphenyl)-1-methylethyl]-2-hydroxy-2-(3-trifluoromethylphenyl)ethanamine hydrochloride-(R,R) isomer.

5. A process for producing a compound of formula (II) or a salt thereof, as defined in claim 1, which process comprises reducing an oxo-group and/or a double bond in a compound of formula (III):

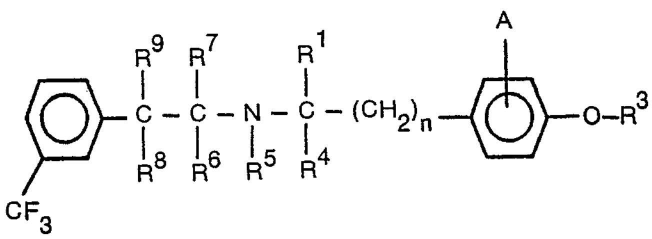

(III)

wherein $R^1$, $R^3$ A and n are as defined in relation to formula (II);

$R^4$ is a group $R^2$ as defined in relation to formula (II) or together with $R^5$ forms a bond;

$R^5$ is hydrogen or together with $R^4$ or $R^6$ forms a bond;

$R^6$ is hydrogen or together with $R^7$ forms an oxo roup or together with $R^5$ forms a bond;

$R^7$ is hydrogen or together with $R^6$ forms an oxo-group;

$R^8$ is hydroxyl or together with $R^9$ forms an oxo-group;

$R^9$ is hydrogen or together with $R^8$ forms an oxo-group, provided that there is no more than one bond represented by any of $R^4$ to $R^9$, and optionally thereafter forming a salt of the compound of formula (II) so formed.

6. A process for producing a compound of formula (II) or a salt thereof, as defined in claim 1, which process comprises reacting a compound of formula (IV):

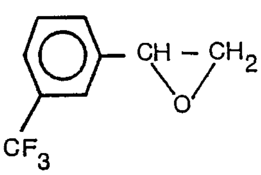

(IV)

with a compound of formula (V):

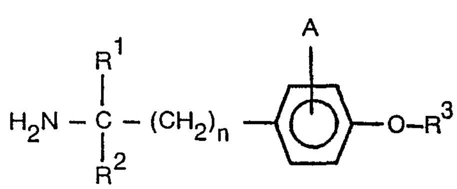

(V)

wherein $R^1$, $R^2$, $R^3$, A and n are as defined in relation to formula (II), and optionally thereafter forming a salt of the compound of formula (II) so formed.

7. A pharmaceutical composition comprising a compound of formula (II), as defined in any one of claims 1 to 4, or a pharmaceutically acceptable salt thereof, in combination with a pharmaceutically acceptable salt thereof, in combination with a pharmaceutically acceptable carrier therefor.

8. A compound of formula (II) as defined in any one of claims 1 to 4, or a pharmaceutically acceptable salt thereof, for use in the treatment of obesity, diabetes, inflammation or platelet aggregation in human or non-human animals.

**Patentansprüche:**

1. Eine Verbindung der Formel (II)

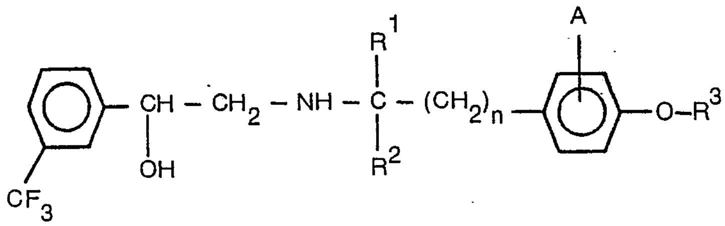

(II)

oder ein Salz derselben,
wobei

$R^1$ Wasserstoff oder Methyl,

$R^2$ Wasserstoff oder Methyl,

$R^3$Wasserstoff, ein $C_{1-12}$-Alkyl, $C_{1-10}$-Cycloalkyl oder Benzyl, gegebenenfalls substituiert mit einem $C_{1-6}$-Alkyl, $C_{1-6}$Alkoxy oder einem Halogen, bedeutet;

n den Wert 1 oder hat;

und

A Wasserstoff, ein Halogen, Hydroxyl, $C_{1-6}$-Alkyl oder $C_{1-6}$-Alkoxy bedeutet.

2. Eine Verbindung gemäß Anspruch 1, in welcher $R^3$ Wasserstoff, $C_{1-6}$-Alkyl oder Benzyl, gegebenenfalls substituiert mit $C_{1-6}$-Alkyl, $C_{1-6}$-Alkoxy oder einem Halogen, bedeutet.

3. Eine Verbindung gemäß Anspruch 1 oder 2, in welcher $R^1$ Wasserstoff und $R^2$ Methyl ist.

4. Eine Verbindung gemäß Anspruch 1, ausgewählt aus

N-[2-(4-Benzyloxyphenyl)-1-methyläthyl]-2-hydroxy-2-(3-trifluormethylphenyl)äthanamin,
N-[2-(4-Hydroxyphenyl)-1-methyläthyl]-2-hydroxy-2-(3-trifluormethylphenyl)äthanamin,
N-[2-(3,4-Dimethoxyphenyl)-1-methyläthyl]-2-hydroxy-2-(3-trifluormethylphenyl)äthanamin,
N-[2-(3-Fluor-4-methoxyphenyl)-1-methyläthyl]-2-hydroxy-2-(3-trifluormethylphenyl)äthanamin,
N-[2-(4-Methoxyphenyl)-1-methyläthyl]-2-hydroxy-2-(3-trifluormethylphenyl)äthanamin,
N-[2-(4-Benzyloxy-3-methoxyphenyl)-1-methyläthyl]-2-hydroxy-2-(3-trifluormethylphenyl)äthanamin,
N-[2-(4-Benzyloxy-3-chlorphenyl)-1-methyläthyl]-2-hydroxy-2-(3-trifluormethylphenyl)äthanamin,
N-[2-(3-Chlor-4-hydroxyphenyl)-1-methyläthyl]-2-hydroxy-2-(3-trifluormethylphenyl)äthanamin,
N-[2-(3-Fluor-4-methoxyphenyl)-1-methyläthyl]-2-hydroxy-2-(3-trifluormethylphenyl)äthanamin-Hydrobromid-(RR,SS) Diastereoisomer,
N-[2-(4-$^n$Decyloxyphenyl)-1-methyläthyl]-2-hydroxy-2-(3-trifluormethylphenyl)äthanamin,
N-[2-(4-$^n$Pentyloxyphenyl)-1-methyläthyl]-2-hydroxy-2-(3-trifluormethylphenyl)äthanamin,
N-[2-(4-Isopropoxyphenyl)-1-methyläthyl]-2-hydroxy-2-(3-trifluormethylphenyl)äthanamin,
N-[2-(4-Methoxyphenyl)äthyl]-2-hydroxy-2-(3-trifluormethylphenyl)äthanamin,
N-[3-(4-Methoxyphenyl)-1,1-dimethylpropyl]-2-hydroxy-2-(3-trifluormethyl)äthanamin, oder
N-[2-(4-Methoxyphenyl)-1-methyläthyl]-2-hydroxy-2-(3-trifluormethylphenyl)äthanamin-Hydrochlorid-(RR) Isomer.

5. Ein Verfahren zur Herstellung einer Verbindung der Formel (II) oder eines Salzes derselben, wie in Anspruch 1 definiert, welches Verfahren die Reduktion einer Oxo-Gruppe und/oder einer Doppelbindung in einer Verbindung der Formel (III):

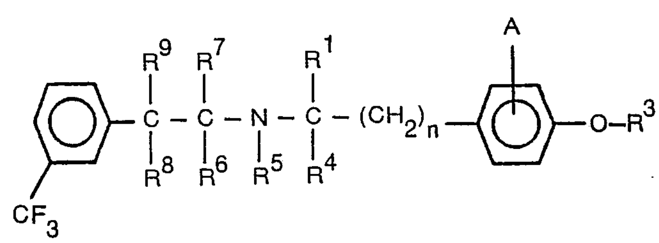

(III)

in welcher $R^1$, $R^3$, A und n wie in bezug auf Formel (II) definiert sind;

$R^4$ eine Gruppe $R^2$ bedeutet, wie in bezug auf Formel (II) definiert, oder zusammen mit $R^5$ eine Bindung bildet.

$R^5$ Wasserstoff ist, oder zusammen mit $R^4$ oder $R^6$ eine Bindung bildet;

$R^6$ Wasserstoff ist, oder zusammen mit $R^7$ eine Oxogruppe bildet oder zusammen mit $R^5$ eine Bindung bildet.

$R^7$ Wasserstoff ist, oder zusammen mit $R^6$ eine Oxogruppe bildet;

$R^8$ Hydroxyl bedeutet, ode zusammen mit $R^6$ eine Oxogruppe bildet;

$R^9$ Wasserstoff ist, oder zusammen mit $R^8$ eine Oxogruppe bildet, vorausgesetzt, daß nur eine Bindung, dargestellt durch eine der Gruppen $R^4$ bis $R^9$, vorhanden ist, und gegebenenfalls anschliessend das Bilden eines Salzes der so gebildeten Verbindung der Formel (II), umfaßt.

6. Ein Verfahren zur Herstellung einer Verbindung der Formel (II) oder eines Salzes derselben, wie in Anspruch 1 definiert, welches Verfahren die Umsetzung einer Verbindung der Formel (IV):

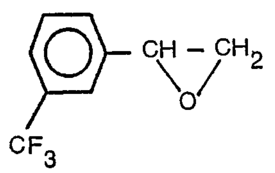

(IV)

mit einer Verbindung der Formel (V):

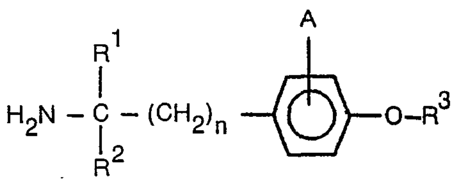

(V)

in welcher $R^1$, $R^2$, $R^3$, A und n wie in bezug auf Formel (II) definiert sind, und gegebenenfalls anschliessend die Bildung eines Salzes der so gebildeten Verbindung der Formel (II) umfaßt.

7. Eine pharmazeutisches Zusammensetzung enthaltend eine Verbindung der Formel (II), wie in irgendeinem der Ansprüche 1 bis 4 definiert, oder ein pharmazeutisch verträgliches Salz derselben, in Verbindung mit einem pharmazeutischen verträglichen Träger.

8. Eine Verbindung der Formel (II) wi in irgendeinem der Ansprüche 1 bis 4 beansprucht, oder ein pharmazeutisch verträgliches Salz derselben, zur Verwendung bei der Behandlung von Fettsucht, Diabetes, Entzündungen oder Thrombosen bei Menschen und tierischen Lebewesen.

**Revendications:**

1. Composé représenté par la formule (II) ciaprès:

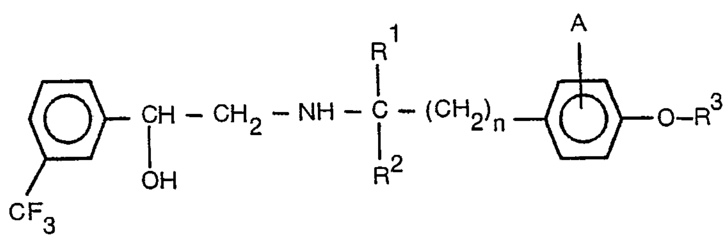

(II)

ou un sel de celui-ci,
dans laquelle

$R^1$ est un atome d'hydrogène ou un groupe méthyle,

$R^2$ est un atome d'hydrogène ou un groupe méthyle,

$R^3$ est un atome d'hydrogène, un groupe alcoyle en $C_{1-12}$, cycloalcoyle en $C_{1-10}$ ou benzyle éventuellement substitué par un groupe alcoyle en $C_{1-6}$, alcoxy en $C_{1-6}$ ou un atome d'halogène,

n est 1 ou 2,

et

A est un atome d'hydrogène, d'halogène, un groupe hydroxy, alcoyle en $C_{1-6}$ ou alcoxy $C_{1-6}$.

2. Composé suivant la revendication 1, caractérisé en ce que $R^3$ est un atome d'hydrogène, un groupe alcoyle en $C_{1-6}$, ou benzyle éventuellement substitué avec un groupe alcoyle en $C_{1-6}$, alcoxy en $C_{1-6}$ ou un atome d'halogène.

3. Composé suivant la revendication 1 ou la revendication 2, caractérisé en ce que $R^1$ est un atome d'hydrogène et $R^2$ est un groupe méthyle.

4. Composé suivant la revendication 1, caractérisé en ce qu'il est choisi parmi la
la N-[2-(4-benzyloxyphényl-1-méthyléthyl]-2-hydroxy-2-(3-trifluorométhylphényl)éthanamine,
la N-[2-(4-hydroxyphényl)-1-méthyléthyl]-2-hydroxy-2-(3-trifluorométhylphényl)éthanamine,
la N-[2-(3,4-diméthoxyphényl)-1-méthyléthyl]-2-hydroxy-2-(3-trifluorométhylphényl)éthanamine,
la N-[2-(3-fluoro-4-méthoxyphényl)-1-méthyléthyl]-2-hydroxy-2-(3-trifluorométhylphényl)éthanamine,
la N-[2-(4-méoxyphényl)-1-méthyléthyl]-2-hydroxy-2-(3-trifluorométhylphényl)éthanamine,
la N-[2-(4-benzyloxy-3-méthoxyphényl)-1-méthyléthyl]-2-hydroxy-2-(3-trifluorométhylphényl)éthanamine,
la N[2-(4-benzyloxy-3-chlorophényl)-1-méthyléthyl]-2-hydroxy-2-(3-trifluorométhylphényl)éthyanamine,
la N-[2-(3-chloro-4-hydroxyphényl)-1-méthyléthyl]-2-hydroxy-2-(3-trifluorométhylphényl)éthanamine,
le diastéréisomère (RR,SS) de bromhydrate de N-[2-(3-fluoro-4-méthoxyphényl)-1-méthyléthyl]-2-hydroxy-2-(3-trifluorométhylphényl)éthanamine,
la N[2-(4-n-décyloxyphényl)-1-méthyléthyl]-2-hydroxy-2-(3-trifluorométhylphényl)éthanamine,
la N-[2-(4-pentyloxyphényl)-1-méthyléthyl]-2-hydroxy-2-(3-trifluorométhylphényl)éthanamine,
la N-[2-(4-isopropoxyphényl)-1-méthyléthyl]-2-hydroxy-2-(3-trifluorométhylphényl)éthanamine,
la N-[2-(4-méthoxyphényl)éthyl]-2-hydroxy-2-(3-trifluorométhylphényl)éthanamine,
la N-[3-(4-méthoxyphényl)-1,1-diméthylpropyl]-2-hydroxy-2-(3-trifluorométhylphényl)éthanamine
ou l'isomère R,R du chlorhydrate de N-[2-(4-méthoxyphényl)-1-méthyléthyl]-2-hydroxy-2-(3-trifluorométhylphényl)éthanamine.

5. Procédé de fabrication d'un composé de formule (II) ou d'un sel de celui-ci suivant la revendication 1, caractérisé en ce qu'il comprend la réduction d'un groupe oxo et/ou d'une double liaison dans un composé de formule (III) ci-aprés:

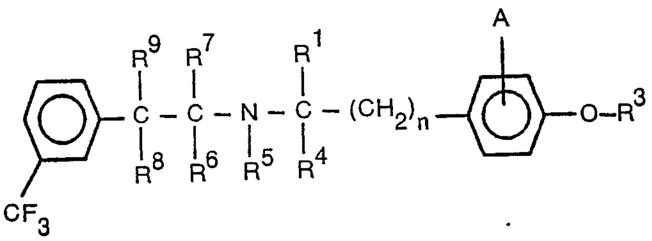
(III)

dans laquelles $R^1$, $R^3$, A et n sont tels que définis à propos de la formule (II),
$R^4$ est un groupe $R^2$ tel que défini à propos de la formule (II), ou forme une liaison avec $R^5$,
$R^5$ est un atome d'hydrogène ou forme une liaison avec $R^4$ ou $R^6$,
$R^6$ est un atome d'hydrogène ou forme un groupe oxo avec $R^7$ ou une liaison avec $R^5$,
$R^7$ est un atome d'hydrogène ou forme un groupe oxo avec $R^6$,
$R^8$ est un groupe hydroxy ou forme un groupe oxo avec $R^9$,
$R^9$ est un atome d'hydrogène ou forme un groupe oxo avec $R^8$, a la condition qu'il n'y ait pas plus d'une liaison représententée par l'un quelconque des radicaux $R^4$ à $R^9$, et ensuite éventuellement, la formation d'un sel du composé de formule (II) ainsi obtenu.

6. Procédé de fabrication d'un composé de formule (II) ou d'un sel de celui-ci suivant la revendication 1, caractérisé en ce qu'il comprend la réaction d'un composé de formule (IV) ci-après:

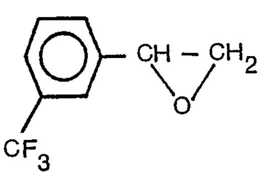
(IV)

avec un composé de formule (V) ci-après:

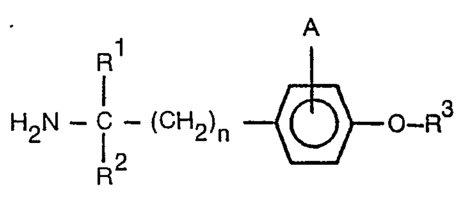
(V)

dans laquelle $R^1$, $R^2$, $R^3$, A et n sont tels que définis à propos de la formule (II) et éventuellement, la formation ultérieure d'un sel du composé de formule (II) ainsi obtenu.

7. Composition pharmaceutique, caractérisée en ce qu'elle comprend un composé de formule (II) tel que défini suivant l'une quelconque des revendications 1 à 4, ou un sel acceptable du point de vue pharmaceutique de celui-ci, en association avec un support pour celui-ci, acceptable du point de vue pharmaceutique.

8. Composé de formule (II) tel que défini suivant l'une quelconque des revendications 1 à 4 ou un sel acceptable du point de vue pharmaceutique de celui-ci, caractérise en ce qu'il est utilisé dans le traitement de l'obésité, du diabetes, de l'inflammation ou de l'agrégation des plaquettes chez l'homme ou un animal non-humain.